(19) Europäisches Patentamt
European Patent Office
Office européen des brevets

(11) **EP 2 206 759 B1**

(12) **EUROPEAN PATENT SPECIFICATION**

(45) Date of publication and mention
of the grant of the patent:
**05.09.2012 Bulletin 2012/36**

(51) Int Cl.:
*C09J 7/02* (2006.01)     *C09J 7/04* (2006.01)
*A61F 13/02* (2006.01)     *A61K 9/70* (2006.01)

(21) Application number: **10250054.3**

(22) Date of filing: **13.01.2010**

(54) **Adhesive preparation and patch preparation**

Klebstoffherstellung und Pflasterherstellung

Préparation adhésive et préparation de timbre

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **13.01.2009 JP 2009004948**

(43) Date of publication of application:
**14.07.2010 Bulletin 2010/28**

(73) Proprietor: **Nitto Denko Corporation**
**Ibaraki-shi**
**Osaka 567-8680 (JP)**

(72) Inventors:
• **Ameyama, Satoshi**
**Ibaraki-shi**
**Osaka 567-8680 (JP)**

• **Inosaka, Keigo**
**Ibaraki-shi**
**Osaka 567-8680 (JP)**

(74) Representative: **Duckworth, Timothy John**
**J A Kemp**
**14 South Square**
**Gray's Inn**
**London WC1R 5JJ (GB)**

(56) References cited:
| | |
|---|---|
| EP-A1- 1 181 930 | WO-A1-2006/104941 |
| WO-A1-2007/140785 | WO-A2-2008/115371 |
| DE-U1- 20 205 521 | DE-U1- 29 615 131 |
| DE-U1-202004 010 911 | US-A1- 2004 071 918 |

**Description**

TECHNICAL FIELD OF THE INVENTION

The present invention relates to an adhesive preparation and a patch preparation, which are easily adhered to the skin and easily handled.

BACKGROUND OF THE INVENTION

Conventionally, adhesive preparations to be adhered to the skin to protect affected parts, and the like, and patch preparations for transdermal administration of a drug on adhesion to the skin surface are widely used.

Adhesive preparations generally have an adhesive preparation main body consisting of a support such as a plastic film and the like and an adhesive layer laminated on the support, and normally further have a release liner laminated on the surface opposite to the support of the adhesive layer.

Conventionally, users of adhesive preparations detach a release liner from an adhesive preparation with fingers when in use, and adheres the adhesive preparation main body to the skin surface. However, since the adhesive preparation and the release liner laminated on each other have the same shape and the same size, detachment of the release liner is not easy for the users, and an adhesive surface (surface of an adhesive layer which comes into contact with the skin) often needs to be touched with fingers to detach the release liner.

An adhesive surface once touched by fingers loses adhesive force somewhat, and phenomena such as falling off of the adhesive preparation main body from a skin surface and edge lifting of an end portion of the adhesive preparation main body may occur upon adhesion of the adhesive preparation main body to the skin surface.

To deal with such problem, JP-A-1997-235220 discloses a multi-layer film preparation to be adhered to an affected part in the mouth cavity for the treatment or protection, which has a part of a release paper protruding in a tab-like form from the preparation, wherein the protruding part facilitates detachment of the release paper. However, since such multi-layer film preparation mainly consists of a water-soluble polymer, it has a certain level of rigidity before containing water, and does not cause any particular difficulty in adhesion in the mouth cavity.

On the other hand, since adhesive preparations and patch preparations are adhered to the skin surface, they are required to follow movements of the skin surface, the adhesive preparation main body to be adhered to the skin surface is formed to have flexibility that allows the preparation to follow active movement of the skin surface. Due to such flexibility, when an adhesive preparation main body after detachment from a release liner is adhered to the skin surface, the adhesive preparation main body sometimes curls up contrary to the intention, or adhesive surfaces may adhere to each other, and therefore, adhesion to the skin surface is not easy even when the aforementioned protruding part is formed on a release liner. As such, a special consideration is necessary for an easily operation of an adhesive preparation or patch preparation.

SUMMARY OF THE INVENTION

The present invention has been made in view of such situation, wherein the problem to be solved is to provide an adhesive preparation or a patch preparation wherein a release liner can be detached easily from an adhesive preparation main body without contact of a finger with the adhesive surface, curling of the adhesive preparation main body and adhesion of different parts of an adhesive surface of the adhesive preparation do not occur, and the adhesive preparation main body can be adhered to the skin surface easily without contact of a finger with the adhesive surface.

The present inventors have conducted intensive studies and found that the aforementioned problems can be solved by an adhesive preparation comprising an adhesive preparation main body comprising a support and an adhesive layer laminated on at least one surface of the support, and a release liner formed on a surface on the side opposite from the support side of the adhesive layer of the adhesive preparation main body, wherein a flat plane area of the adhesive preparation main body and a ratio of a flat plane area of the release liner to that of the adhesive preparation main body are set to fall within particular ranges, and the release liner has a separating line, which resulted in the completion of the present invention.

Accordingly, the present invention provides an adhesive preparation as defined in claim 1.

WO 2007/140785 A1 discloses a mounting pad comprising an adhesive layer provided with a removable release liner divided into at least two sections.

In the adhesive preparation of the present invention, since the flat plane area of the adhesive preparation main body is 1.5 - 15 cm$^2$, and the ratio of the flat plane area of the adhesive preparation main body to the flat plane area of the release liner ([flat plane area of release liner (cm$^2$)]/[flat plane area of adhesive preparation main body (cm$^2$)]) is 1.1 - 7, the adhesive preparation main body can be easily held with fingers, and the release liner can secure a holding section having a sufficient area. Therefore, the release liner can be easily detached from the adhesive preparation main body without touching the adhesive surface with fingers and the adhesive preparation main body can be easily adhered to the skin, whereby inconveniences such as mutual adhesion of a certain part of the adhesive surface to other part, curling of the adhesive preparation main body and the like can be prevented.

In addition, when the release liner has a separating line that divides the release liner into two or more plural release liner fragments, one of the plurally-divided release liner fragments (preferably a release liner fragment with a smaller area when one separating line produces two release liner fragments) is first detached from the adhesive preparation main body, the exposed adhesive surface of the adhesive preparation main body is partially adhered and fixed onto the skin, then the remaining release liner fragment is detached, and the adhesive preparation main body can be adhered to the skin. Therefore, the release liner can be detached from the adhesive preparation main body more easily without touching the adhesive surface with fingers, and the adhesive preparation main body can be adhered to the skin more easily without touching the adhesive surface with fingers.

In addition, since the release liner has a separating line, the entire adhesive surface of the adhesive preparation main body can be partially exposed rather than all at once. As a result, inconveniences such as mutual adhesion of a certain part of the adhesive surface to other part, curling of the adhesive preparation main body and the like can be further prevented.

Using the adhesive preparation of the present invention, moreover, the release liner can be detached from the adhesive preparation main body with ease without touching the adhesive surface with fingers and the adhesive preparation main body can be adhered to the skin with ease without touching the adhesive surface with fingers. Therefore, reduction of adhesive force of the adhesive surface by touching the adhesive surface with fingers before adhesion to the skin (affected part) can be suppressed, and the possibility of the adhesive preparation main body adhered to the skin falling off therefrom and edge lifting of the adhesive preparation main body adhered to the skin can be reduced.

The adhesive preparation of the present invention can contain a drug in the adhesive layer, and can provide a patch preparation to be adhered to the skin surface for transdermal administration of the drug. Generally, when the adhesive layer of an adhesive preparation does not contain water, the adhesive force of the adhesive surface markedly decreases by touching the adhesive surface once with fingers. In addition, when fingers touch an adhesive surface of a patch preparation containing a drug in the adhesive layer during adhesion of the patch preparation, components such as sebum and the like and components attached to the fingers may be blended into the adhesive layer. Such components can cause chemical changes in the drug contained in the adhesive layer, and produce an adverse influence on the drug efficacy. Therefore, the adhesive preparation of the present invention not requiring contact with the adhesive surface during adhesion is particularly effective when the adhesive layer does not contain water in the adhesive layer and the adhesive layer contains a drug.

BRIEF DESCRIPTION OF THE DRAWINGS

Fig. 1 is a perspective view (A) and a sectional view (B) schematically showing the adhesive preparation of a first example, useful for an understanding of the present invention.

Fig. 2 is a perspective view (A) and a sectional view (B) schematically showing the adhesive preparation of a second example, according to an embodiment of the present invention.

Fig. 3 is a perspective view (A) and a sectional view (B) schematically showing the adhesive preparation of a third example, according to an embodiment of the present invention.

Figs. 4(A) - (D) explains the relative configurational relationship between the adhesive preparation main body and the release liner in the flat plane view of the adhesive preparation of the present invention.

In the Figures, 1 is a release liner, 2 is an adhesive layer, 3 is a support, 4 is an adhesive preparation main body (patch preparation main body), 11 is a separating line, 1A is a release liner fragment with large flat plane area, 1B is a release liner fragment with a small flat plane area, 1a is a release liner protrusion part (holding section), and W is the width of the narrowest protrusion part (holding section) of the release liner.

DETAILED DESCRIPTION OF THE INVENTION

In the present specification, the adhesive preparation is a concept including not only those without containing a drug but also those containing a drug. Those containing a drug are sometimes particularly referred to as patch preparations.

The present invention is explained in the following by referring to the attached Figures and according to preferable embodiments. The following detailed explanations and particular examples do not limit the scope of the present invention. The attached Figures are schematic showings, and the size of each constituent element and the size ratio of different constituent elements in the Figures are not necessarily the same as those in the product of the present invention.

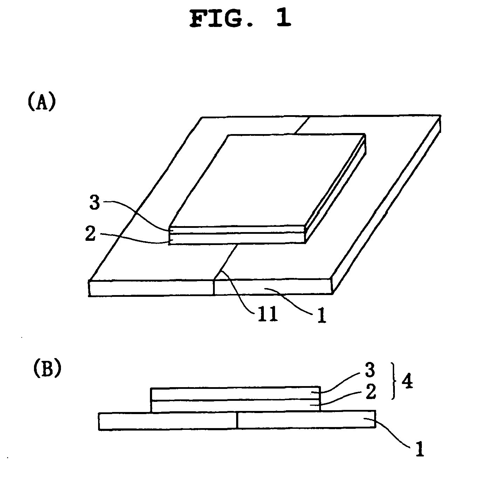

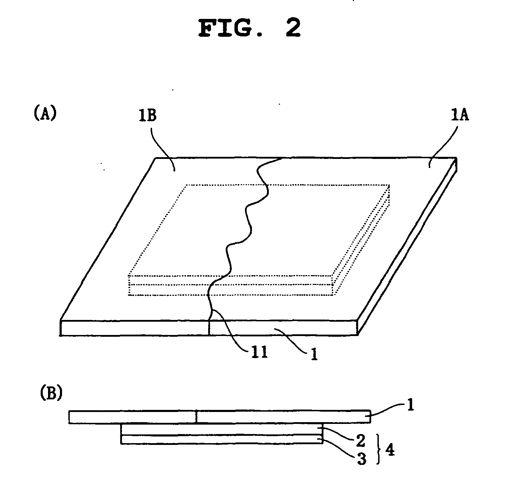

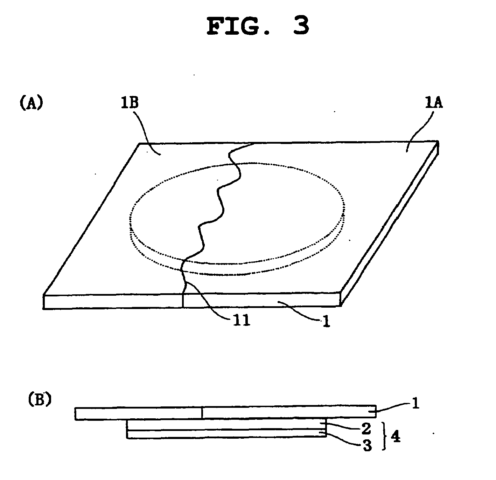

Fig. 1 schematically shows an example useful for an understanding of the present invention. Fig. 2 and Fig. 3 schematically show the first embodiment and the second embodiment of the adhesive preparation of the present invention, wherein Figure (A) in each of Figs. 1 - 3 shows a perspective view, and Figure (B) shows a sectional view. In Fig. 1, the adhesive preparation main body 4 is disposed at the upper side of the drawing, and a release liner 1 at the lower side of the drawing. In Figs. 2, 3, the adhesive preparation main body 4 is disposed at the lower side of the drawing, and the release liner 1 at the upper side of the drawing.

As shown in the adhesive preparations of the first embodiment and the second embodiment (Fig. 2 and Fig. 3), the adhesive preparation of the present invention comprises an adhesive preparation main body 4 comprising a support 3 and an adhesive layer 2 laminated on at least one surface of the support 3, and a release liner 1 formed on a surface on the side opposite from the support 3 side of the adhesive layer 2 of the adhesive preparation main body 4, wherein

the adhesive preparation main body 4 has a flat plane area of 1.5 - 15 cm$^2$, and a ratio of a flat plane area of the release liner to that of the adhesive preparation main body 4 ([flat plane area (cm$^2$) of release liner]/[flat plane area (cm$^2$) of adhesive preparation main body 4]) is 1.1 - 7, and

the release liner 1 has a separating line 11.

In the present invention, the "flat plane area of the adhesive preparation main body" can be shown by an "area of the support". To be specific, an adhesive layer to be formed on at least one surface of the support is generally formed on the entire area of one surface of the support, and therefore, the surface area of the adhesive layer to be the main surface of one adhesive preparation main body is substantially the same as the area of the support.

In the present specification, when the adhesive preparation main body contains a drug in the adhesive preparation, it may sometimes be particularly referred to as a patch preparation main body.

In the adhesive preparation of the present invention, the flat plane area of the adhesive preparation main body is set to fall within the range of 1.5 - 15 cm$^2$. When the adhesive preparation has such area, the adhesive preparation main body can be easily held with fingers. In addition, when the surface (the adhesive surface) of the adhesive layer in the adhesive preparation main body is exposed, mutual adhesion of a certain part of the adhesive surface to other part becomes difficult, and curling of the adhesive preparation main body does not occur easily. Thus, the adhesive preparation main body can be easily adhered to the skin without touching the adhesive surface with fingers.

When the flat plane area of the adhesive preparation main body is less than 1.5 cm$^2$, the adhesive preparation main body cannot be easily held with fingers, and detachment of the release liner and adhesion of the adhesive preparation main body to the skin tend to be difficult. When the flat plane area of the adhesive preparation main body is more than 15 cm$^2$, inconveniences such as mutual adhesion of a certain part of the exposed adhesive surface of the adhesive preparation main body to other part thereof, curling of the adhesive preparation main body and the like tend to occur.after detachment of the release liner. As a result, adhesion of the adhesive preparation main body to the skin becomes difficult.

In the adhesive preparation of the present invention, the ratio of a flat plane area of the release liner to that of the adhesive preparation main body ([flat plane area (cm$^2$) of release liner]/[flat plane area (cm$^2$) of adhesive preparation main body]) is set to fall within the range of 1.1 - 7. Within this range, the release liner can secure a holding section having a sufficient area, the release liner can be easily detached from the adhesive preparation main body without touching the adhesive surface (a surface of the adhesive layer to be in contact with the skin) with fingers, and the adhesive preparation main body can be easily adhered to the skin without touching the adhesive surface with fingers.

When the aforementioned ratio is less than 1.1, the characteristic of improved adhesion operability is not observed. On the other hand, when the aforementioned ratio exceeds 7, the stress is focused on the adhesive layer of the adhesive preparation main body at the part where the adhesive surface is detached from the release liner during the detachment of the release liner. As a result, after detachment of the release liner, the support may curl with the adhesive surface facing inside to make the adhesion operation difficult to perform.

In the adhesive preparation of the present invention, the lower limit of the flat plane area of the adhesive preparation main body is preferably not less than 2 cm$^2$, more preferably not less than 2.5 cm$^2$. The upper limit of the flat plane area of the adhesive preparation main body is preferably not more than 12.5 cm$^2$, more preferably not more than 12 cm$^2$, particular preferably not more than 10 cm$^2$.

In addition, the lower limit of the ratio of the flat plane area of the release liner to that of the adhesive preparation main body ([flat plane area (cm$^2$) of release liner]/[flat plane area (cm$^2$) of adhesive preparation main body]) is preferably not less than 1.35. The upper limit of the aforementioned ratio is preferably not more than 6, more preferably not more than 3.

When the flat plane area of the adhesive preparation main body and the ratio of the flat plane area of the release liner to that of the adhesive preparation main body are within the above-mentioned preferable ranges, the release liner can be detached from the adhesive preparation main body more easily without touching the adhesive surface with fingers, and the adhesive preparation main body can be adhered to the skin more easily without touching the adhesive surface with fingers.

As mentioned above, important characteristics of the adhesive preparation of the present invention are that the flat plane area of the adhesive preparation main body is set to fall within a particular range and the flat plane area of the release liner is greater within a particular range than the flat plane area of the adhesive preparation main body. Consequently, the adhesive preparation main body is easily held with fingers, and the release liner can secure a holding section having a sufficient area. Thus, the release liner can be easily detached from the adhesive preparation main body without touching the adhesive surface with fingers, and the adhesive preparation main body can be easily adhered to the skin without touching the adhesive surface with fingers. In addition, inconveniences such as mutual adhesion of a certain part of the adhesive surface to other part thereof, curling of the adhesive preparation main body and the like can be prevented.

Moreover, since in the adhesive preparation of the present invention enables detachment of a release liner from the adhesive preparation main body and adhesion of the adhesive preparation main body to the skin, without contact of a finger with the adhesive surface of the adhesive preparation main body, reduction of adhesive force of the adhesive surface by touching the adhesive surface with fingers before adhesion to the skin (affected part) can be suppressed, and the possibility of the adhesive preparation main body adhered to the skin falling off therefrom and edge lifting of the adhesive preparation main body adhered to the skin can be also decreased.

In the adhesive preparation of the present invention, the shape of the flat plane of the adhesive preparation main body and the shape of the flat plane of the release liner are not particularly limited, and they may take any form as long as detachment of the release liner, adhesion of the adhesive preparation main body and the like are not affected. Examples of the shape of the flat plane of the adhesive preparation main body and release liner include a shape outlined by about straight lines such as polygon (e.g., triangle, quadrangle, pentagon and the like), and the like, a shape outlined by a curve such as ellipse, circular shape and the like, a shape outlined by both about straight lines and curve and the like. The about straight lines here are concept include not only perfect straight line but also lines recognized as straight lines as a whole, which may contain an ultrafine meander portion visually not recognizable with ease.

Examples of the preferable embodiment of the shape of the flat plane of the adhesive preparation main body and the shape of the flat plane of the release liner include the embodiment of Fig. 2, wherein both of the shape of the flat plane of the adhesive preparation main body and the shape of the flat plane of the release liner are rectangular or about rectangular, the embodiment of Fig. 3 wherein the shape of the flat plane of the adhesive preparation main body is a circular shape or ellipse, and the shape of the flat plane of the release liner is rectangular or about rectangular, and the like, from the aspects of detachability of the release liner, the adhesion of the adhesive preparation main body, easiness of the production of the adhesive preparation and the like.

The "rectangular" in the present invention is a concept including rectangle and square, and the "about rectangular" means a quadrangle generally recognized as a rectangle even if all four angles of the quadrangle are not strictly at a right angle.

Particularly, an embodiment wherein the shape of the flat plane of the adhesive preparation main body is outlined by a curve such as ellipse, a circular shape and the like, and the shape of the flat plane of the release liner is rectangular or about rectangular is preferable. The curve is optionally substituted in part by a straight line. In other words, an embodiment wherein the shape of the flat plane of the adhesive preparation main body is outlined mainly by a curve, and the shape of the flat plane of the release liner is rectangular or about rectangular is preferable. In such embodiment, since the holding section of the release liner becomes particularly large at each corner of the rectangular or about rectangular release liner, the release liner can be detached from the adhesive preparation main body particularly easily without touching the adhesive surface with fingers, and the adhesive preparation main body can be adhered to the skin particularly easily without touching the adhesive surface with fingers. The shape outlined mainly by a curve is not limited to a shape outlined only by a curve, such as ellipse, circular shape and the like, but is a concept including a shape outlined by a curve in the main part but also outlined in part by a straight line, such as oval and the like.

In the adhesive preparation of the present invention, the release liner has a separating line. In the adhesive preparations of the first example - the third example shown in Fig. 1 - Fig. 3, a single separating line 11 is formed in a release liner 1.

The separating line in the release liner is a split to divide the release liner into two or more plural release liner fragments, and mostly provided to divide the release liner into two fragments, sometimes into 3 or more fragments. In the present invention, the number of the separating lines is not particularly limited. Generally, as in the adhesive preparations of the first example - the third example shown in Fig. 1 - Fig. 3, a single separating line 11 is provided to divide the release liner into two fragments. While the separating line is generally formed to completely cut the release liner, it may be formed such that one end of the separating line stops before the outer circumference of the release liner. In addition, the cut line may be a broken line (perforated line) rather than a complete cut line.

The shape of the separating line (cut line) is not particularly limited, and may be a straight line as shown in Fig. 1, wavy lines shown in Fig. 2 and Fig. 3, hook-like line, convex concave line etc., or two or more of the exemplified lines may be mixed. To provide a clue to the position of the separating line, and facilitate noting of the separating line, a wavy line is preferable.

The separating line is preferably a single line dividing the flat plane area of the adhesive preparation main body into nonuniform two, as in the adhesive preparations of the first embodiment and the second embodiment shown in Fig. 2 and Fig. 3, that is, dividing the adhesive preparation main body into a first adhesive preparation main body (with a smaller area of adhesive preparation main body) and a second adhesive preparation main body (with a larger area of area of adhesive preparation main body). More preferably, the separating line is a single separating line present at a position where a division ratio of the adhesive preparation main body divided into two by the separating line ([area of larger adhesive preparation main body]/[area of smaller adhesive preparation main body]) is greater than 1.0 and not more than 4.0. In such embodiment, the area and the adhesive force of the first adhesive surface are sufficiently ensured by (a) first detaching the first release liner fragment corresponding to the first adhesive preparation main body, and adhering the exposed first adhesive surface to the skin; and then (b) detaching the second release liner fragment corresponding to the second adhesive preparation main body while the first adhesive surface is adhered to the skin, and adhering the exposed second adhesive surface to the skin. Thus, when the second release liner fragment is detached while the first adhesive surface is adhered to the skin, the first adhesive preparation main body does not need to be pressed sufficiently with finger during the detachment. In addition, since the adhesive preparation main body with a smaller area is first adhered to the skin, the adhesive preparation main body with a larger area can function as a holding section during adhesion of the adhesive preparation main body to the skin. Hence, the adhesive preparation main body can be adhered to the skin particularly easily without touching the adhesive surface with fingers.

Examples of other preferable embodiment of the separating line include adhesive preparations of first and second embodiments shown in Fig. 2 and Fig. 3, wherein a release liner 1 is divided into two fragments of release liner fragment 1A with a larger flat plane area and a release liner fragment 1B with a smaller flat plane area. The ratio of the area of one release liner fragment to that of the other release liner fragment ([area of larger release liner fragment]/[area of smaller release liner fragment]) is preferably greater than 1.0 and not more than 4.0, more preferably not less than 1.1 and not more than 3.0. In such embodiment, when the adhesive preparation main body is adhered to the skin, of the two release liner fragments divided by the separating line, release liner fragment 1A with a larger flat plane area is held by hands and release liner fragment 1B with a smaller flat plane area is detached, the exposed adhesive surface of the adhesive preparation main body is adhered to the skin, and then, the release liner fragment 1A with a larger flat plane area is detached, and the adhesive preparation main body can be further adhered to the skin. That is, the release liner fragment to be held by the hand can be enlarged than in the embodiment where a separating line is formed to dividing the release liner into two release liner fragments with an equal area. Therefore, the release liner can be detached from the adhesive preparation main body more easily without touching the adhesive surface with fingers, and the adhesive preparation main body can be adhered to the skin more easily without touching the adhesive surface with fingers.

In addition, in an embodiment where a separating line in the release liner divides the adhesive preparation main body into a first adhesive preparation main body (with a smaller area of adhesive preparation main body) and a second adhesive preparation main body (with a larger area of adhesive preparation main body), a first release liner fragment corresponding to the first adhesive preparation main body (with a smaller area of the adhesive preparation main body) may be a release liner fragment with a smaller area or a release liner fragment with a larger area. However, since the release liner fragment to be held by hands has a larger flat plane area, in a preferable embodiment, the first release liner fragment is a release liner fragment with a smaller area.

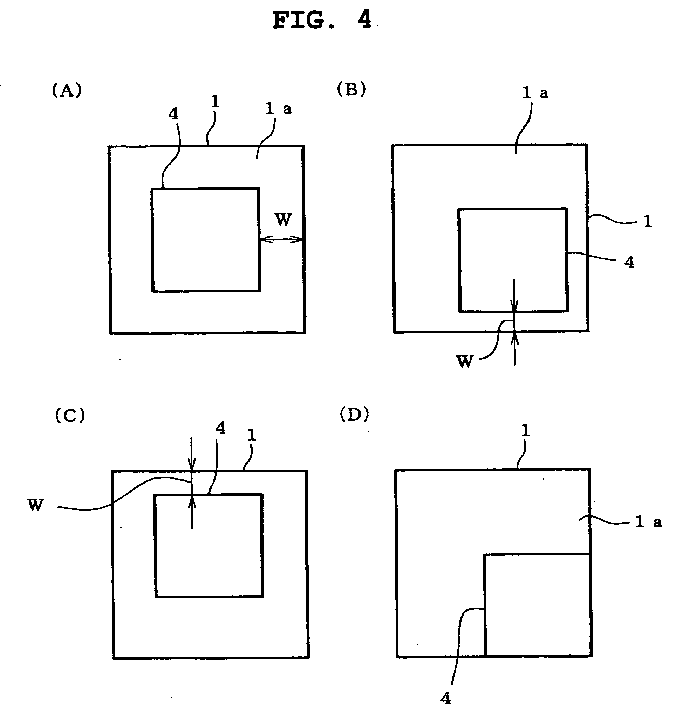

The adhesive preparation of the present invention is not particularly limited as to the relative configurational relationship between the adhesive preparation main body and the release liner in the flat plane view. For example, Figs. 4(A) - (D) schematically show the adhesive preparation of the present invention, wherein the shape of the flat plane of the adhesive preparation main body 4 is rectangular and the shape of the flat plane of the release liner 1 is rectangular. The adhesive preparation of the present invention may have a constitution as shown in Fig. 4(A) wherein the adhesive preparation main body 4 and release liner 1 are concentrically configured (i.e., the center point of the flat plane of the adhesive preparation main body 4 and that of the flat plane of the release liner 1 are superimposed), or a constitution as shown in Fig. 4 (B) - Fig. 4(D) wherein the adhesive preparation main body 4 and release liner 1 are eccentrically configured (i.e., the center point of the flat plane of the adhesive preparation main body 4 and that of the flat plane of the release liner 1 are separated). From the aspects of detachability of the release liner, adhesion of the adhesive preparation main body, suppression of attachment of an adhesive layer component of the adhesive preparation to a packaging material due to extrusion of an adhesive layer and the like, a configuration as shown in Figs. 4(A) - (C), wherein a protrusion (holding section) 1a of release liner 1 is formed on the whole circumference of the adhesive preparation main body 4 is preferable. In this case, the width W of the protrusion (holding section) with the minimum width is more preferably not less than 0.5 mm.

In the adhesive preparation of the present invention, the support of the adhesive preparation main body is not particularly limited, and a film-like or a sheet-like material known per se is used. Such support is preferably one substantially impermeable to the components in an adhesive layer such as drugs, additives and the like, namely, one that does not allow them to pass through the support and get lost from the back face of the adhesive preparation to lower the content.

Examples of such support include polyester such as poly(ethylene terephthalate) and the like, resin single layer film such as nylon, saran (registered trade mark of Asahi Kasei Corporation, The Dow Chemical Company, US), polyethylene, polypropylene, polyvinyl chloride, ethylene-ethyl acrylate copolymer, polytetrafluoroethylene, surlyn (registered trade mark of E. I. du Pont de Nemours and Company US) and the like, a single layer film such as metal foil and the like, a laminate film thereof, a laminate of a porous solid on the single layer or laminate film and the like.

The aforementioned porous solid may be a single layer or a laminate, and one having an anchor ability to suppress movement of the adhesive layer relative to the porous solid can be preferably used. Specific examples thereof include paper, woven fabric, non-woven fabric, knitted fabric, a mechanically perforated film, a laminate thereof and the like. Of these, from the aspects of handling property and the like, particularly preferred are paper, woven fabric, non-woven fabric and a laminate thereof. Of these, non-woven fabric is preferable.

The support may appropriately contain, as long as the effect of the present invention is not impaired, various additives such as antioxidant, pigment, antistatic agent and the like, and may be subjected to a surface treatment such as corona discharge treatment, UV-irradiation treatment and the like. The thickness of the film in the support (when the support is a laminate with a porous solid, the thickness of the film excluding the porous solid) is preferably within the range of 1 - 18 $\mu$m from the aspects of the skin following property of the adhesive preparation, and adhesive operability and the like. When the thickness is less than 1 $\mu$m, the function as a support of a flexible adhesive layer may be insufficient. When it is not more than 18 $\mu$m, the support develops curl upon detachment of the release liner. Therefore, when the thickness of the film is not more than 18 $\mu$m, the advantages of the present invention become particularly noticeable.

When the support is a laminate with a porous solid, the present inventors consider that the above-mentioned property of the support is controlled by the film property.

The film thickness of the support (when the support is a laminate with a porous solid, the film thickness excludes the porous solid) can be measured by, after staining the adhesive preparation with, for example, aqueous ruthenium acid solution, cutting the adhesive preparation with a freezing microtome, imaging the sectional surface with FE-SEM (Hitachi, S-4800) at x50 - 1000 magnification, and reading the gauge scale. The thickness of the adhesive preparation main body (total of the thicknesses of the support and the adhesive layer) is preferably within the range of 46 - 500 $\mu$m, in consideration of the adhesion operability, the skin following property and the like.

Now, a method for preparing a composition for forming the adhesive layer (hereinafter to be also referred to as a composition for forming an adhesive layer or a coating solution), and the amount of components constituting the adhesive layer are explained. The content ratios (amounts) of respective components described in the following, which are contained in the composition for forming the adhesive layer, show the proportions of the amount of each component relative to the total amount of all components excluding a solvent (organic solvent) in wt% unit.

The adhesive layer can be formed by mixing an adhesive with a drug, tackifier, organic liquid component and the like as necessary in the presence of an organic solvent to give a composition for forming an adhesive layer, forming a layer thereof by a method such as coating and the like and drying the layer.

While the organic solvent is not limited, one compatible with the aforementioned respective components constituting the adhesive layer, which can be volatilized easily in a drying step, is preferable. Examples of such organic solvent include aromatic hydrocarbons such as toluene, xylene and the like, aliphatic hydrocarbon such as hexane and the like, esters such as ethyl acetate and the like, alcohols such as ethanol and the like, ethers such as diethyl ether, tetrahydrofuran etc., and the like. These may be used singly or in combination of two or more kinds thereof.

The aforementioned drying may be performed by air-drying, or according to a known method using a dryer, hot air, far infrared rays and the like.

While the mixing method of the aforementioned respective components is not limited, kneading machines such as a kneader, a planetary mixer and the like, dispersing machines such as a homogenizer and the like, stirring machines such as propeller mixer etc., and the like can be mentioned. These can be used singly or in combination of two or more kinds thereof.

While the adhesive to be used for the adhesive layer is not particularly limited, acrylic adhesives (acrylic polymer); styrene-diene-styrene block copolymers (e.g., styreneisoprene-styrene block copolymer, styrene-butadiene-styrene block copolymer etc.); rubber adhesives such as polyisoprene, polyisobutylene, polybutadiene and the like; silicone adhesives such as silicone rubber, dimethylsiloxane base, diphenylsiloxane base and the like; vinyl ether adhesives such as polyvinyl methylether, polyvinyl ethylether, polyvinyl isobutylether and the like; vinyl ester adhesives such as vinyl acetate-ethylene copolymer and the like; polyester adhesives consisting of a carboxylic acid component such as dimethyl terephthalate, dimethyl isophthalate, dimethyl phthalate and the like and a polyvalent alcohol component such as ethylene glycol and the like, and the like can be mentioned.

The adhesive layer may be a crosslinked adhesive layer after a crosslinking treatment, or may be a non-crosslinked adhesive layer free of a crosslinking treatment. Here, the crosslinking treatment refers to a known treatment applied to an adhesive layer in an attempt to simultaneously achieve sufficient maintenance of the skin adhesive force of an adhesive preparation, and low stimulation to the skin caused by pulling the skin for peeling off the adhesive preparation from the skin surface, and physically scraping the stratum corneum layer from the skin, and examples of the crosslinking treatment include a chemical crosslinking treatment, an ion crosslinking treatment, and a physical crosslinking treatment by electron beam, ultraviolet and the like. Examples of the crosslinking agent include metal salts such as zinc acetate and the like, epoxy compound, amide compound, amine compound, acid anhydride, peroxide, isocyanate compound and the like.

The adhesive layer is preferably a hydrophobic adhesive layer from the aspect of the skin adhesiveness. Here, the "hydrophobic adhesive layer" means an adhesive layer having a water concentration of not more than 5 wt%, and the above-mentioned water concentration means a value measured according to the Karl-Fisher method.

A non-water-containing adhesive layer markedly loses the adhesive force after adhesion to the skin once, and sometimes fails to provide a sufficient adhesive force when adhered again. Therefore, when fingers and the like contacts the adhesive surface during the adhesion operation, the adhesive preparation main body tends to easily fall off from the skin surface after the adhesion, and edge lifting of an end portion of the adhesive preparation main body after the adhesion and the like tend to occur. Hence, the adhesive preparation of the present invention which does not require contact with the adhesive surface during detachment of the release liner and adhesion of the adhesive preparation main body can be particularly advantageously practiced when the adhesive layer is a non-water-containing adhesive layer.

Moreover, an adhesive layer using a rubber adhesive or an acrylic adhesive cannot be detached easily when the adhesive surfaces of an adhesive layer adheres to each other, and an adhesive layer using a rubber adhesive tends to lose adhesive force of the adhesive surface when the adhesive surface touches the skin. Hence, the adhesive preparation of the present invention which does not require contact with the adhesive surface during detachment of the release liner and adhesion of the adhesive preparation main body can be particularly advantageously practiced when the adhesive layer contains a rubber adhesive or an acrylic adhesive.

As a rubber adhesive, a mixture of those having the same or different components and different average molecular weights can be used to achieve a suitable adhesive force and suitable drug dissolution property. Taking polyisobutylene for example, a mixture of high molecular weight polyisobutylene having a viscosity average molecular weight of 1,800,000 - 5,500,000, middle molecular weight polyisobutylene having a viscosity average molecular weight of 40,000 - 85,000, and, where necessary, low molecular weight polyisobutylene is preferable. The viscosity average molecular weight in the present invention is obtained by calculating the Staudinger index ($J_0$) according to the Schulz-Blaschke equation from the flow time of capillary 1 of the Ubbelohde's viscometer at 20°C, and applying the $J_0$ value to the following equations.

$$J_0 = \eta_{sp}/\{c(1+0.31\eta_{sp})\} \text{ (Schulz-Blaschke equation}$$

$$\eta_{sp} = t/t_0 - 1$$

t: flow time of solution (according to Hagenbach-couette correction)
$t_0$: flow time of solvent (according to Hagenbach-couette correction)
c: concentration of solution (g/cm$^3$)

$$J_0 = 3.06 \times 10^{-2} Mv^{0.65}$$

Mv: viscosity average molecular weight

Here, it is preferable to contain high molecular weight polyisobutylene in a proportion of 10 - 80 wt%, preferably 10 - 50 wt%, middle molecular weight polyisobutylene in a proportion of 0 - 90 wt%, preferably 10 - 80 wt%, and low molecular weight polyisobutylene in a proportion of 0 - 80 wt%, preferably 0 - 60 wt%.

An adhesive layer using a rubber adhesive may contain, for example, a tackifier such as rosin resin, polyterpene resin, coumarone-indene resin, petroleum resin, terpene-phenol resin, xylene resin and the like to achieve suitable adhesiveness. These are used singly or in a mixture of two or more kinds thereof. Examples of the aforementioned petroleum resin include alicyclic saturated hydrocarbon resins obtained by partially or completely hydrogenated aliphatic (C5) petroleum resin, aromatic (C9) petroleum resin, copolymerized (C5-C9) petroleum resin and aromatic (C9) petroleum resin. As the alicyclic saturated hydrocarbon resin, one having a softening point (ring and ball method) of 90 - 150°C is preferable. While the amount of the tackifier to be blended is not limited, it is, for example, 10 - 40 wt% from the aspects of imparting suitable adhesiveness and preventing saturation of the effect of the tackifier when the amount thereof is increased.

The thickness of the adhesive layer is preferably 20 - 190 $\mu$m to ensure followability of the adhesive preparation main body to the skin movement, desired skin adhesive force and the like.

When desired, the adhesive layer can contain an organic liquid component. The organic liquid component is not particularly limited, and glycols such as ethylene glycol, diethylene glycol, propylene glycol, dipropylene glycol, tripropylene glycol, triethylene glycol, polyethylene glycol, polypropylene glycol and the like; fats and oils such as olive oil, castor oil and the like; lanolin; hydrocarbons such as squalene, liquid paraffin; various surfactants; ethoxylated stearyl alcohol; glycerol monoesters such as oleic acid monoglyceride, caprylic acid monoglyceride, lauryl acid monoglyceride; dialkyl esters of polyalkylene glycol such as polypropylene glycol; glycerol diesters such as glycerol diacetate and the like, glycerol triesters such as glycerol triacetate and the like, or a mixture thereof; fatty acid alkylesters such as triethyl citrate and the like; long chain alcohol; higher fatty acids such as oleic acid, caprylic acid; alkylesters of higher fatty acid such as isopropyl myristate; pyrrolidones such as N-methylpyrrolidone and N-dodecylpyrrolidone; sulfoxides such as decylmethyl sulfoxide; 1,3-butanediol and the like can be mentioned. These can be used singly or in a mixture of two or more kinds thereof. The organic liquid component can be preferably contained in a proportion of 0 - 60 wt%, more preferably 0 - 50 wt%.

When desired, the adhesive preparation of the present invention can contain a drug in the adhesive layer, and can provide a patch preparation. The drug herein is not particularly limited, and a drug that can be administered to a mammal such as human and the like through the skin thereof, i.e., a transdermally absorbable drug, is preferable.

Specific examples of such drug include general anesthetics, hypnotic sedatives, antiepileptic drugs, antipyretic analgesic antiphlogistic drugs, anti-vertiginous drugs, psychoneurotic drugs, topical anesthetics, skeletal muscle relaxants, autonomic drugs, antiepileptic drugs, anti-parkinsonian drugs, anti-histamine drugs, cardiac stimulants, drugs for arrhythmia, diuretic, hypotensive drug, vasoconstrictor, coronary vasodilator, peripheral vasodilators, arteriosclerosis drugs, drugs for circulatory organ, anapnoics, antitussive expectorant, hormone drugs, external drugs for mattery diseases, analgesic-antipruritic-styptic-antiphlogistic drugs, drugs for parasitic dermatic diseases, drugs for arrest of bleeding, gout treatment drugs, drugs for diabetes, drugs for anti-malignant tumor, antibiotic, chemical therapy drugs, narcotic, quit smoking aids and the like.

While the content of the drug is not particularly limited as long as the effect by transdermal absorption is provided and adhesive property of the adhesive is not impaired, it is preferably 0.1 - 60 wt%, more preferably 0.1 - 40 wt%. When the content is less than 0.1 wt%, the treatment effect may be insufficient. When it is more than 60 wt%, skin irritation may be developed and economical disadvantage may be caused.

When fingers touch an adhesive surface during adhesion of a patch preparation, components such as sebum and the like and components attached to the fingers may be blended into the adhesive layer. Such components can cause chemical changes in the drug contained in the adhesive layer, and produce an adverse influence on the drug efficacy. Therefore, the adhesive preparation of the present invention not requiring contact with the adhesive surface during adhesion can be particularly advantageously practiced as a patch preparation containing a drug in the adhesive layer.

In the adhesive preparation of the present invention, a material of the release liner to be provided on the adhesive surface of the adhesive preparation main body is not particularly limited, and those known in the art can be used without limitation. Specific examples thereof include plastic films of polyesters such as poly(ethylene terephthalate), poly(vinyl chloride), poly(vinylidene chloride), various acrylic-based and methacrylic-based polymers, polystyrene, polycarbonate, polyimide, acetyl cellulose, regenerated cellulose (cellophane), celluloid and the like, a laminate film of high-quality paper, glassine paper and the like and polyolefin and the like. In view of safety, economic aspect and drug transfer, a polyester film is preferably used.

The thickness of the release liner is preferably about 25 - 100 $\mu$m to maintain a given shape of a flexible adhesive preparation main body, facilitate holding of the adhesive preparation and the like.

The release liner is preferably treated for easy peeling on the interfacial surface side with an adhesive layer, so as to facilitate peeling from the adhesive layer. While the easy peeling treatment is not limited, a known method can be applied. For example, a treatment for forming a peeling-treated layer using a release agent comprising a curable silicone resin as a main component by a coating method such as bar coating, gravure coating and the like can be applied. The thickness of the peeling-treated layer is preferably 0.01 - 5 $\mu$m to ensure release property and uniformity of the coating.

As the production method of the adhesive preparation and patch preparation of the present invention, various methods are available. However, for industrial production, for example, the following method is preferable for high production efficiency.

A composition for forming an adhesive layer is applied to at least one surface of a release liner and dried to give the adhesive layer. Then, a support is laminated thereon to give a sheet for adhesive preparation production (when the adhesive layer contains a drug, it is hereinafter to be also referred to as a sheet for patch preparation production).

Alternatively, a composition for forming an adhesive layer is applied to at least one surface of a support, and dried to give an adhesive layer. A release liner is laminated thereon to give a sheet for adhesive preparation production.

A method for the above-mentioned lamination is not particularly limited, and a known means such as coating, adhesion, fusion bonding, melt bonding, pressure bonding and the like of a primer and the like can be employed.

Then, one or more separating lines are formed on a release liner of a sheet for adhesive preparation production. A method for forming a separating line is not limited, and any known cutting method can be used. For example, a separating line can be formed by completely cutting substantially a release liner alone with a film-cutting cutter to form a wavy line from the release liner side (opposite side from adhesive layer) of a sheet for adhesive preparation production, at a position that divides the flat plane area of the adhesive preparation main body into nonuniform two. The position of the separating line can be controlled as appropriate, and the number and shape of the separating line can be determined as appropriate. A method for forming a separating line by partial cutting is not limited and, for example, a circle cutter with partial blades set on the circumference can be used by rotation.

Thereafter, the adhesive preparation main body consisting of a support and an adhesive layer alone from the support side is cut into a given shape such as rectangle, circle, ellipse and the like (what is called island cut), whereby the adhesive preparation main body having a flat plane area of 1.5 - 15 cm$^2$ can be formed. While a method for island cut is not limited, a press blade that provides a flat plane area of the adhesive preparation main body of 1.5 - 15 cm$^2$ is preferably used, since the flat plane area, cutting size, and sectional shape can be easily adjusted, alignment is easy and a clean end surface'can be obtained. Such island cut can be performed, for example, on a flat table while controlling the gap between the press blade and the table during cutting.

Lastly, the release liner is cut at a predetermined position such that the ratio of the flat plane area of the release liner to that of the adhesive preparation main body is 1.1 - 7, whereby an adhesive preparation (patch preparation when the adhesive layer contains a drug) can be obtained. The cutting can be performed in such a manner that, for example, the release liner will have an about rectangular shape of the flat plane. A method for the cutting is not limited, and any known cutting method such as laser, press blade and the like can be used.

Examples

The present invention is explained in more detail in the following by referring to Examples and Comparative Examples, which are not to be construed as limitative.

Examples 1 - 17

<Preparation of composition for forming adhesive layer (coating solution)>

A solution (about 8 wt%, 313 g) of high molecular weight polyisobutylene (viscosity average molecular weight $4 \times 10^6$) in toluene was measured, a solution (about 55 wt%, 64 g) of medium molecular weight polyisobutylene (viscosity average molecular weight $5.5 \times 10^4$) in toluene, a tackifier (alicyclic saturated hydrocarbon resin, softening point 141°C (ring and ball method), 20 g) and scopolamine (5 g) as a drug were added thereto, and the mixture was stirred. Then, liquid paraffin (15 g) as an organic liquid component and toluene (209 g) as an organic solvent were further added, and the mixture was sufficiently stirred to give a uniform coating solution.

<Production of sheet for patch preparation production>

The coating solution was applied to an easy peeling surface of a polyethylene terephthalate (hereinafter to be also referred to as "PET") release liner (thickness 75 $\mu$m) subjected to an easy peeling treatment such that the thickness of the adhesive layer after drying was 100 $\mu$m, dried by a dryer (100°C) to give a release liner with an adhesive layer. The adhesive surface (surface on which an adhesive layer is formed) was press-adhered to a laminate as a support of 2 $\mu$m-thick PET film and a PET non-woven fabric (fabric weight 12 g/m$^2$) to give a sheet for the production of the patch preparation. The adhesive layer of the obtained sheet for the production of the patch preparation was of a water-non-containing type which contains a drug but is substantially free of water. In addition, the water concentration of the adhesive layer was not more than 0.5 wt% according to the Karl-Fisher method.

<Production of patch preparation>

The release liner of the sheet for the production of the patch preparation obtained as mentioned above was completely cut with an edged tool from the side opposite from the adhesive layer at the position at which the area of the patch preparation main body of the patch preparation to be obtained later is nonuniformly divided at about 1:1.5, and a wavy separating line (one) was formed. Using a press blade having a predetermined shape (rectangular, circular or ellipse) and capable of punching processing into a predetermined shape (rectangular, circular or ellipse), only the patch preparation main body consisting of the support and the adhesive layer was cut from the support side into a predetermined shape on a flat table after adjusting the gap between the press blade and the table during cutting (i.e., island cut) to give a rectangular part as shown in Fig. 2 or circular or ellipse part as shown in Fig. 3 of the patch preparation main body. The patch preparation main body was cut (island cut) such that the flat plane area of the patch preparation main body was 2 - 10 cm$^2$. Lastly, release liner was cut into a rectangle such that the ratio of a flat plane area of the release liner to that of the adhesive preparation main body ([flat plane area (cm$^2$) of release liner]/[flat plane area (cm$^2$) of adhesive preparation main body]) was 1.2 - 4 to give a patch preparation of the embodiment shown in Fig. 2 or Fig. 3, wherein the release liner and the patch preparation main body are set substantially concentrically.

The shape and flat plane area of the patch preparation main body, and the ratio of the flat plane area of the release liner to that of the adhesive preparation main body in each Example are shown in Table 1, and the shape and the size of the patch preparation main body in each Example are shown in Table 2. Hereinafter the flat plane area in Table 1 and the like is also simply indicated as area.

Comparative Examples 1 - 26

In the same manner as in Examples 1 - 17 except that the patch preparation main body was cut (island cut) such that the shape of the flat plane and size of the patch preparation main body were as shown in Table 2, and the patch preparation main body was cut (island cut) and release liner was cut such that the flat plane area of the patch preparation main body and the ratio of the flat plane area of the release liner to that of the adhesive preparation main body were as shown in Table 1, a coating solution was prepared, a sheet for the production of a patch preparation was produced, and a patch preparation was produced.

Examples 18 - 25 and Reference Examples 1 - 6

In the same manner as in Examples 1 - 17 except that the patch preparation main body was cut (island cut) such that the shape of the flat plane and size of the patch preparation main body were as shown in Table 4, and the patch preparation main body was cut (island cut) and release liner was cut such that the flat plane area of the patch preparation main body, the ratio of the flat plane area of the release liner to that of the adhesive preparation main body and the ratio of the areas of the patch preparation main body divided by a separating line were as shown in Table 3, a coating solution was prepared in the same manner as in Examples 1 - 17, a sheet for the production of a patch preparation was produced, and a patch preparation was produced.

[Table 1]

| shape and flat plane area of patch preparation main body, and ratio of flat plane area of release liner to flat plane area of patch preparation main body | | | | | | | |
|---|---|---|---|---|---|---|---|
| patch preparation main body | | [area (cm$^2$) of release liner]/[area (cm$^2$) of patch preparation main body] | | | | | |
| shape | area (cm$^2$) | 1.0 | 1.2 | 1.5 | 2 | 4 | 8 |
| rectangle | 1.0 | Comp. Ex. 1 | Comp. Ex. 2 | Comp. Ex. 3 | Comp. Ex. 4 | Comp. Ex. 5 | Comp. Ex. 6 |
| | 2.0 | Comp. 7 Ex. 7 | Ex. 1 | Ex. 2 | Ex. 3 | Ex. 4 | Comp. Ex. 8 |
| | 5.0 | Comp. 9 Ex. 9 | Ex. 5 | Ex. 6 | Ex. 7 | Ex. 8 | Comp. Ex. 10 |
| | 10 | Comp. 11 Ex. 11 | Ex. 9 | Ex. 10 | Ex. 11 | Ex. 12 | Comp. Ex. 12 |
| | 20 | Comp. Ex. 13 | Comp. Ex. 14 | Comp. Ex. 15 | Comp. Ex. 16 | Comp. Ex. 17 | Comp. Ex. 18 |
| ellipse | 10 | Comp. Ex. 19 | - | Ex. 13 | Ex. 14 | Ex. 15 | Comp. Ex. 20 |
| | 20 | Comp. Ex. 21 | - | - | Comp. Ex. 22 | Comp. Ex. 23 | Comp. Ex. 24 |
| circle | 10 | Comp. Ex. 25 | - | - | Ex. 16 | Ex. 17 | Comp. Ex. 26 |

[Table 2]

Shape of patch preparation main body (size (mm))

| Ex. 1 – 4 | rectangle (10×20) | Comp. Ex. 1 – 6 | rectangle (10×10) |
|---|---|---|---|
| Ex. 5 – 8 | rectangle (20×25) | Comp. Ex. 7, 8 | rectangle (10×20) |
| Ex. 9 – 12 | rectangle (25×40) | Comp. Ex. 9, 10 | rectangle (20×25) |
| Ex. 13 – 15 | ellipse (major axis 20, minor axis 16) | Comp. Ex. 11, 12 | rectangle (25×40) |
| Ex. 16, 17 | circle | Comp. Ex. 13 – 18 | rectangle (40×50) |
| | | Comp. Ex. 19, 20 | ellipse (major axis 20, minor axis 16) |
| | | Comp. Ex. 21 – 24 | ellipse (major axis 30, minor axis 21) |
| | | Comp. Ex. 25, 26 | circle |

[Table 3]

| flat plane area of patch preparation main body, and ratio of flat plane area of release liner to flat plane area of patch preparation main body and ratio of areas of patch preparation main body divided by separating line | | | | | | | | |
|---|---|---|---|---|---|---|---|---|
| area (cm$^2$) of patch preparation main body | area ratio | ratio of areas of patch preparation main body divided by separating line | | | | | | |
| | | 1.0 | 1.1 | 1.5 | 2.0 | 3.0 | 5.0 | 10.0 |
| 5.0 | 1.5 | Ref. Ex. 1 | Ex. 18 | Ex. 19 | Ex. 20 | Ex. 21 | Ref. Ex. 2 | Ref. Ex. 3 |
| 10 | 2 | Ref. Ex. 4 | Ex. 22 | Ex. 23 | Ex. 24 | Ex. 25 | Ref. Ex. 5 | Ref. Ex. 6 |
| 1) The "area ratio" is the ratio of flat plane area of the release liner to the flat plane area of the patch preparation main body ([flat plane area (cm$^2$) of release liner]/[flat plane area of patch preparation main body (cm$^2$)]). 2) The "ratio of areas of patch preparation main body divided by separating line" is [area (cm$^2$) of larger patch preparation main body]/[area (cm$^2$) of smaller patch preparation main body]. | | | | | | | | |

[Table 4]

| shape of patch preparation main body (size (mm)) | | | |
|---|---|---|---|
| Ex. 18 - 21 | rectangle (20×25) | Ref. Ex. 1 - 3 | rectangle (20×25) |
| Ex. 22 - 25 | ellipse (major axis 20, minor axis 16) | Ref. Ex. 4 - 6 | ellipse (major axis 20, minor axis 16) |

Experimental Example 1 (evaluation of detachability of release liner and easy adhesion of patch preparation)

Expert five raters evaluated the patch preparations of Examples 1 - 17 and Comparative Examples 1 - 26 shown in Table 1 and Table 2. For evaluation, a release liner fragment having a smaller flat plane area was first detached, the patch preparation main body was partially adhered to the skin starting from the exposed adhesive surface, then a release liner fragment having a larger flat plane area was detached, the patch preparation main body was further adhered to the skin, and rated by giving 1 to 4 evaluation points according to the following evaluation criteria. The results are shown by evaluation points rounded to an integer closest to the average of the evaluation points of the raters obtained by evaluating 43 kinds of patch preparations of Examples 1 - 17 and Comparative Examples 1 - 26.

<Evaluation criteria>

1: Release liner can be extremely easily detached from patch preparation main body without touching adhesive surface with fingers, and patch preparation main body can be extremely easily adhered to the skin without touching adhesive surface with fingers.
2: Either release liner can be extremely easily detached from patch preparation main body without touching adhesive surface with fingers, or patch preparation main body can be extremely easily adhered to the skin without touching adhesive surface with fingers, and the other is possible.
3: Detachment of release liner from patch preparation main body without touching adhesive surface with fingers is somewhat difficult, and/or adhesion of patch preparation main body to the skin without touching adhesive surface with fingers is somewhat difficult.
4: Detachment of release liner from patch preparation main body without touching adhesive surface with fingers is difficult, and/or adhesion of patch preparation main body to the skin without touching adhesive surface with fingers is difficult.

The results of Experimental Example 1 are shown in Table 5. As is clear from the results of Table 5, when the ratio of the flat plane area of the release liner to that of the adhesive preparation main body was 1.2 - 4 and the flat plane area of the patch preparation main body was 2.0 - 10 $cm^2$, detachment of the release liner and adhesion of the patch preparation posed no problem, and at least either the release liner could be extremely easily detached from the patch preparation main body without touching the adhesive surface with fingers, or the patch preparation main body could be extremely easily adhered to the skin without touching the adhesive surface with fingers, and the other is possible. The patch preparation main body did not curl during adhesion, and mutual adhesion of the adhesive surfaces did not occur. Furthermore, when the ratio of the flat plane area of the release liner to that of the adhesive preparation main body was 1.5 - 2 and the flat plane area of the patch preparation main body was 5.0 $cm^2$ or above, the release liner could be extremely easily detached from the patch preparation main body without touching the adhesive surface with fingers and the patch preparation main body could be extremely easily adhered to the skin without touching the adhesive surface with fingers. Therefore, the operability of the patch preparation was particularly fine.
In Examples 13 - 17, since the shape of the flat plane of the patch preparation main body was circular or ellipse, the holding section of the release liner became particularly large at each corner of the rectangular release liner and the periphery of the patch preparation main body, which particularly facilitated detachment of the release liner, and the operability during adhesion was particularly fine.
[Table 5]

| results of evaluation of patch preparations in Experimental Example 1 | | | | | | | |
|---|---|---|---|---|---|---|---|
| patch preparation main body | | [area ($cm^2$) of release liner]/[area ($cm^2$) of patch preparation main body] | | | | | |
| shape | area ($cm^2$) | 1.0 | 1.2 | 1.5 | 2 | 4 | 8 |
| rectangle | 1.0 | 4 | 4 | 4 | 3 | 3 | 4 |
| | 2.0 | 4 | 2 | 2 | 2 | 2 | 4 |
| | 5.0 | 3 | 2 | 1 | 1 | 2 | 4 |
| | 10 | 3 | 2 | 1 | 1 | 2 | 4 |
| | 20 | 3 | 3 | 3 | 4 | 4 | 4 |
| ellipse | 10 | 3 | - | 1 | 1 | 2 | 4 |
| | 20 | 3 | - | - | 3 | 4 | 4 |
| circle | 10 | 3 | - | - | 1 | 1 | 4 |

Experimental Example 2 (evaluation of detachment.of release liner fragment to be detached later and adhesion of part of patch preparation main body to be adhered earlier)

Expert five raters evaluated the patch preparations of Examples 18 - 25 and Reference Examples 1 - 6 shown in Table 3 and Table 4. For evaluation, from among the release liner fragments obtained by division with a separating line present at the position at which the area of the patch preparation main body is nonuniformly divided, a release liner fragment having a smaller flat plane area was first detached, the patch preparation main body was partially adhered to the skin starting from the exposed adhesive surface, then a release liner fragment having a larger flat plane area was detached, the patch preparation main body was further adhered to the skin, and rated by giving 1 to 3 evaluation points according to the following evaluation criteria. To be specific, whether the larger release liner fragment can be detached without causing detachment of a part of the patch preparation main body adhered earlier was evaluated. In addition, before adhesion to the skin, the degree of curl of a part of the patch preparation main body corresponding to the smaller release liner fragment detached earlier was also evaluated visually. The results are shown by evaluation points rounded to an integer closest to the average of the evaluation points of the raters obtained by evaluating 14 kinds of patch preparations of Examples 18 - 25 and Reference Examples 1 - 6.

<Evaluation criteria>

1: Release liner fragment to be detached later can be easily detached even without pressing with finger a part of patch preparation main body adhered earlier.
2: Release liner fragment to be detached later can be detached when a part of patch preparation main body adhered earlier is pressed with finger.
3: Part of patch preparation main body corresponding to smaller release liner fragment detached earlier is somewhat curled before adhesion to the skin.

The results of Experimental Example 2 are shown in Table 6. As shown in Table 6, when the flat plane area of the patch preparation main body, and the ratio of a flat plane area of the release liner to that of the adhesive preparation main body are constant, the release liner fragment to be detached later could be easily detached even without pressing with finger a part of the patch preparation main body adhered earlier, since a separating line was present at the position at which the flat plane area of the patch preparation main body was divided at a division ratio of 1.1 - 3.0, and the operability during adhesion was particularly fine. As compared to such embodiment, when the separating line was present at the position at which the flat plane area of the patch preparation main body was divided at a division ratio of 1.0, a part of the patch preparation main body corresponding to the release liner fragment detached earlier was somewhat curled before adhesion to the skin, and the operability during adhesion was rather inferior.
[Table 6]

| results of evaluation of patch preparations in Experimental Example 2 | | | | | | | | |
|---|---|---|---|---|---|---|---|---|
| area (cm$^2$) of patch preparation main body | area ratio | ratio of areas of patch preparation main body divided by separating line | | | | | | |
| | | 1.0 | 1.1 | 1.5 | 2.0 | 3.0 | 5.0 | 10.0 |
| 5.0 | 1.5 | 3 | 1 | 1 | 1 | 1 | 2 | 2 |
| 10 | 2 | 3 | 1 | 1 | 1 | 1 | 2 | 2 |
| 1) The "area ratio" is the ratio of flat plane area of the release liner to the flat plane area of the patch preparation main body ([flat plane area (cm$^2$) of release liner]/[flat plane area of patch preparation main body (cm$^2$)]).<br>2) The "ratio of areas of patch preparation main body divided by separating line" is [area (cm$^2$) of larger patch preparation main body]/[area (cm$^2$) of smaller patch preparation main body]. | | | | | | | | |

The explanation in the present invention is merely for exemplification purposes, and various modifications and changes to the particular embodiments shown without substantially departing from the teaching and advantages of the present invention are encompassed in the present invention.
The adhesive preparation of the present invention can be preferably used as a medical adhesive preparation for covering and protecting the affected part on the skin surface, and when a drug is contained, it can be used as a patch preparation for continuous transdermal administration of a transdermally absorbable drug. The adhesive preparation of the present invention can be particularly advantageously practiced when the adhesive layer is a non-water-containing adhesive layer and the adhesive layer contains a drug. The adhesive preparation and patch preparation of the present invention can be efficiently produced and are also suitable for industrial production.

In another embodiment, in the adhesive preparation of the present invention the adhesive preparation main body has a flat plane shape substantially outlined by a curve, and the release liner has a rectangular or substantially rectangular flat plane shape.

## Claims

1.  An adhesive preparation comprising an adhesive preparation main body (4) comprising a support (3) and an adhesive layer (2) laminated on at least one surface of the support, and a release liner (1) formed on a surface on the side opposite from the support side of the adhesive layer of the adhesive preparation main body, wherein the release liner has a separating line (11).
    charaterised in that:

    the adhesive preparation main body has a flat plane area of 1.5 - 15 $cm^2$, and a ratio of a flat plane area of the release liner to that of the adhesive preparation main body ([flat plane area ($cm^2$) of release liner]/[flat plane area ($cm^2$) of adhesive preparation main body]) is 1.1 - 7; and
    the separating line is a single line dividing the flat plane area of the adhesive preparation main body into nonuniform two (1A, 1B); and
    a division ratio of the adhesive preparation main body divided into two by the separating line ([area of larger adhesive preparation main body]/[area of smaller adhesive preparation main body]) is larger than 1.0 and not more than 4.0.

2.  An adhesive preparation according to claim 1, wherein the flat plane area of the adhesive preparation main body is not less than 2.5 $cm^2$, and the ratio of a flat plane area of the release liner to that of the adhesive preparation main body ([flat plane area ($cm^2$) of release liner]/[flat plane area ($cm^2$) of adhesive preparation main body]) is not less than 1.35.

3.  An adhesive preparation according to claim 1 or claim 2, wherein the ratio of the area of one release liner fragment to that of the other release liner fragment ([area of larger release liner fragment]/[area of smaller release liner fragment]) is not less than 1.1 and not more than 3.0.

4.  An adhesive preparation according to any one of claims 1 to 3, wherein the adhesive preparation main body has a flat plane shape mainly outlined by a curve, and the release liner has a rectangular or about rectangular flat plane shape.

5.  An adhesive preparation according to any one of claims 1 to 4, which is substantially free of water.

6.  An adhesive preparation according to any one of claims 1 to 5, wherein the adhesive layer comprises a rubber adhesive or acrylic adhesive.

7.  A patch preparation comprising an adhesive preparation according to any one of claims 1 to 6 and a drug contained in the adhesive layer.

## Patentansprüche

1.  Klebepräparat, umfassend einen Hauptkörper (4) des Klebepräparats, der einen Träger (3) und eine Kleberschicht (2), die auf wenigstens eine Fläche des Trägers laminiert ist, umfasst, und eine Schutzfolie (1), die auf einer Fläche auf der Seite gebildet ist, die der Trägerseite der Kleberschicht des Hauptkörpers des Klebepräparats gegenüber liegt, wobei die Schutzfolie eine Trennlinie (11) aufweist,
    **dadurch gekennzeichnet, dass**:

    der Hauptkörper des Klebepräparats einen flachen Ebenenbereich von 1,5 bis 15 $cm^2$ aufweist und das Verhältnis des flachen Ebenenbereichs der Schutzfolie zu dem des Hauptkörpers des Klebepräparats ([flacher

Ebenenbereich (cm$^2$) der Schutzfolie]/[flacher Ebenenbereich (cm$^2$) des Hauptkörpers des Klebepräparats]) 1,1 bis 7 beträgt; und

die Trennlinie eine einzelne Linie ist, die den flachen Ebenenbereich des Hauptkörpers des Klebepräparats in zwei ungleiche Teile (1A, 1B) teilt; und

das Teilungsverhältnis des Hauptkörpers des Klebepräparats, der durch die Trennlinie in zwei Teile geteilt ist ([Fläche des größeren Hauptkörpers des Klebepräparats]/[Fläche des kleineren Hauptkörpers des Klebepräparats]), größer als 1,0 und nicht größer als 4,0 ist.

**2.** Klebepräparat gemäß Anspruch 1, wobei der flache Ebenenbereich des Hauptkörpers des Klebepräparats nicht kleiner als 2,5 cm$^2$ ist und das Verhältnis des flachen Ebenenbereichs der Schutzfolie zu dem des Hauptkörpers des Klebepräparats ([flacher Ebenenbereich (cm$^2$) der Schutzfolie]/[flacher Ebenenbereich (cm$^2$) des Hauptkörpers des Klebepräparats]) nicht kleiner als 1,35 ist.

**3.** Klebepräparat gemäß Anspruch 1 oder 2, wobei das Verhältnis der Fläche des einen Schutzfolienfragments zu der des anderen Schutzfolienfragments ([Fläche des größeren Schutzfolienfragments]/[Fläche des kleineren Schutzfolienfragments]) nicht kleiner als 1,1 und nicht größer als 3,0 ist.

**4.** Klebepräparat gemäß einem der Ansprüche 1 bis 3, wobei der Hauptkörper des Klebepräparats eine flache ebene Form hat, die hauptsächlich von einer Kurve begrenzt ist, und die Schutzfolie eine rechteckige oder etwa rechteckige flache ebene Form hat.

**5.** Klebepräparat gemäß einem der Ansprüche 1 bis 4, das im Wesentlichen frei von Wasser ist.

**6.** Klebepräparat gemäß einem der Ansprüche 1 bis 5, wobei die Kleberschicht einen Kautschukkleber oder Acrylkleber umfasst.

**7.** Pflasterpräparat, das ein Klebepräparat gemäß einem der Ansprüche 1 bis 6 und einen in der Kleberschicht enthaltenen Wirkstoff umfasst.

## Revendications

**1.** Préparation adhésive comprenant un corps principal de préparation adhésive (4) comprenant un support (3) et une couche adhésive (2) stratifiée sur au moins une surface du support, et une pellicule de protection détachable (1) formée sur une surface sur le côté opposé au côté de support de la couche adhésive du corps principal de préparation adhésive, dans laquelle la pellicule de protection détachable présente une ligne de séparation (11), **caractérisée en ce que**

le corps principal de la préparation adhésive présente une surface plane unie de 1,5 - 15 cm$^2$, et un rapport d'une surface plane unie de la pellicule de protection détachable sur celle du corps principal de la préparation adhésive ([surface plane unie (cm$^2$) de la pellicule de protection détachable]/[surface plane unie (cm$^2$) du corps principal de la préparation adhésive]) est de 1,1 à 7 ; et

la ligne de séparation est une ligne simple divisant la surface plane unie du corps principal de la préparation adhésive en deux parties non uniformes (1A, 1B) ; et

un rapport de division du corps principal de la préparation adhésive divisé en deux par la ligne de séparation ([surface du corps principal de la préparation adhésive plus grande]/[surface du corps principal de la préparation adhésive plus petite]) est supérieur à 1,0 et ne dépasse pas 4,0.

**2.** Préparation adhésive selon la revendication 1, dans laquelle la surface plane unie du corps principal de la préparation adhésive n'est pas inférieure à 2,5 cm$^2$, et le rapport de la surface plane unie de la pellicule de protection détachable sur celle du corps principal de la préparation adhésive ([surface plane unie (cm$^2$) de la pellicule de protection détachable]/[surface plane unie (cm$^2$) du corps principal de la préparation adhésive]) n'est pas inférieur à 1,35.

**3.** Préparation adhésive selon la revendication 1 ou la revendication 2, dans laquelle le rapport de la surface d'un fragment de pellicule de protection détachable sur celle de l'autre fragment de pellicule de protection détachable ([surface du fragment de pellicule de protection détachable plus grand]/[surface du fragment de pellicule de protection détachable plus petit]) n'est pas inférieur à 1,1 et pas supérieur à 3,0.

**4.** Préparation adhésive selon l'une quelconque des revendications 1 à 3, dans laquelle le corps principal de la préparation adhésive a une forme plane unie principalement configurée par une courbe, et la pellicule de protection détachable a une forme plane unie rectangulaire ou presque rectangulaire.

5. Préparation adhésive selon l'une quelconque des revendications 1 à 4, qui est sensiblement exempte d'eau.

6. Préparation adhésive selon l'une quelconque des revendications 1 à 5, dans laquelle la couche adhésive comprend un adhésif de caoutchouc ou un adhésif acrylique.

7. Préparation pour timbre comprenant une préparation adhésive selon l'une quelconque des revendications 1 à 6 et un médicament contenu dans la couche adhésive.

# FIG. 1

(A)

(B)

# FIG. 2

(A)

(B)

# FIG. 3

(A)

(B)

# FIG. 4

(A)

(B)

(C)

(D)

**EP 2 206 759 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9235220 A

- WO 2007140785 A1